(19) Europäisches Patentamt
European Patent Office
Office européen des brevets

(11) Veröffentlichungsnummer : **0 052 314**
**B2**

(12) # NEUE EUROPÄISCHE PATENTSCHRIFT

(45) Veröffentlichungstag der neuen Patentschrift : **09.09.87**

(51) Int. Cl.[4] : **C 07 C 41/16**, C 07 C 43/23

(21) Anmeldenummer : **81109542.1**

(22) Anmeldetag : **06.11.81**

(54) **Herstellung von Monoalkyläthern von Hydroxyphenolen.**

(30) Priorität : **15.11.80 DE 3043230**

(43) Veröffentlichungstag der Anmeldung : **26.05.82 Patentblatt 82/21**

(45) Bekanntmachung des Hinweises auf die Patenterteilung : **23.11.83 Patentblatt 83/47**

(45) Bekanntmachung des Hinweises auf die Entscheidung über den Einspruch : **09.09.87 Patentblatt 87/37**

(84) Benannte Vertragsstaaten :
**AT BE CH DE FR GB IT LI NL**

(56) Entgegenhaltungen :
**EP-A- 0 025 843**
**DE-A- 1 806 870**
**DE-A- 2 007 737**
**DE-A- 2 925 763**
**FR-A- 2 255 279**
**GB-A- 2 006 211**
**JP-A-53 059 025**
**US-A- 3 923 901**
**Houben-Weyl VI/3, S. 61, 62, 66, 67**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Büttner, Gerhard, Dr.**
**Adolf-von-Menzel-Strasse 1**
**D-5024 Pulheim (DE)**
Erfinder : **Judat, Artur, Dr.**
**Mozartstrasse 7**
**D-4018 Langenfeld (DE)**
Erfinder : **Allenbach, Udo, Dr.**
**Stammheimer Ring 52**
**D-5000 Köln 80 (DE)**
Erfinder : **Lenthe, Manfred, Dr.**
**Michaelshöhe 40**
**D-5068 Odenthal (DE)**

EP 0 052 314 B2

**Beschreibung**

Die vorliegende Erfindung betrifft ein neues Verfahren zur Monoalkylierung von Brenzkatechin.

Bei der selektiven Monoverätherung von Hydroxyphenolen können eine Reihe von Nebenreaktionen eintreten : Die unerwünschte Diätherbildung vermindert deutlich die Ausbeute an Monoäther ; in Abhängigkeit vom verwendeten Lösungsmittel werden darüber hinaus unterschiedliche Mengen an Kernalkylierungsprodukten erhalten. Will man die Diätherbildung durch nur teilweisen Umsatz des Hydroxyphenols unterdrücken, so muß bei wirtschaftlicher Fahrweise das teure Hydroxyphenol zurückgewonnen werden. Dies ist im allgemeinen nur durch eine aufwendige Extraktion mit großen Lösungsmittelmengen möglich. Eine destillative Wiedergewinnung des eingesetzten Hydroxyphenols führt wegen des hohen Siedepunkts dieser Verbindungen einerseits zu erheblichen Verlusten und andererseits zu kaum wiedereinsatzfähigem Hydroxyphenol, da die gebildeten Kernalkylierungsprodukte einen ähnlichen Siedepunkt wie das Hydroxyphenol haben.

Zur Lösung dieser Probleme bei der Herstellung von Monoäthern aus Hydroxyphenolen sind verschiedene Verfahren vorgeschlagen worden. Es ist bereits nach DOS 2 007 737 bekannt, daß sich Hydroxyphenole mit niedrigen Alkylhalogeniden monoalkylieren lassen, wenn man mit Alkalicarbonaten als Base in einem mit Wasser nicht mischbaren Lösungsmittel bei Temperaturen bis 130 °C und Reaktionszeit von 8-10 Stunden in einer Zweiphasen-Reaktion umsetzt. Als Katalysatoren können tertiäre Amine verwendet werden. Die optimale Wassermenge liegt bei 3-4 Mol Wasser je Mol Hydroxyphenol.

Zur Beschleunigung der Reaktionsgeschwindigkeit setzt man nach einem ähnlichen Verfahren (DOS 2 925 763) anstelle von tertiären Aminen quartäre Ammonium- oder Phosphoniumverbindungen zu, die als Phasentransferkatalysatoren wirken.

In beiden Verfahren wird trotz Zweiphasen-Reaktion und Katalysatoren nur dann eine zufriedenstellende Selektivität für den Monoäther erhalten, wenn der Umsatz an Hydroxyphenol nicht vollständig ist (< 70 %). Für eine wirtschaftliche Herstellung des Monoäthers muß das nicht umgesetzte Hydroxyphenol aus beiden Phasen zurückgewonnen werden, was einen hohen Aufwand erfordert (technisch schwierig) und mit dem eingangs beschriebenen Problem verbunden ist. Wir in einem unpolaren Lösungsmittel ohne Gegenwart von Wasser gearbeitet, ist der Umsatz an Hydroxyphenol völlig unbefriedigend (vgl. Beispiel 7 der DE-OS 2 007 737).

Weiterhin ist in DE-OS 2 451 936 (US-P 3 927 118) ein Verfahren für die selektive Monoalkylierung von Hydroxyphenolen beschrieben. Dabei werden Hydroxyphenole mit Alkylierungsmitteln und Erdalkalihydroxiden als Base in dipolar aprotischen Lösungsmitteln umgesetzt. Nachteilig an diesem Verfahren ist die Aufarbeitung und der doppelte Überschuß an Hydroxyphenol. Die erhaltenen Umsetzungsprodukte (Mono- und etwas Diäther) müssen zunächst durch Extraktion aus dem Reaktionsgemisch entfernt und unter Abtrennung großer Lösungsmittelmengen isoliert werden. Danach kann das nicht umgesetzte Hydroxyphenol und das zur Alkylierung verwendete Lösungsmittel aus der wäßrigen Phase durch eine weitere Destillation erhalten werden (zweiphasige Aufarbeitung).

Die gleichen Probleme und Schwierigkeiten in der Aufarbeitung treten auf, wenn man analog DOS 2 845 429 mit Alkalicarbonaten in dipolar aprotischen Lösungsmitteln umsetzt, ohne allerdings die hohen Brenzkatechinüberschüsse wie in DE-OS 2 451 936 zu verwenden. Es gelingt auch hier nicht, bei einem guten Mono-/ Diäther-Verhältnis Brenzkatechin weitgehend umzusetzen. Beide Verfahren sind außerdem auf die Reaktion mit den reaktionsfähigeren Alkylhalogeniden beschränkt.

Es wurde nun gefunden, daß man Brenzkatechinmonoisopropyläther der Formel

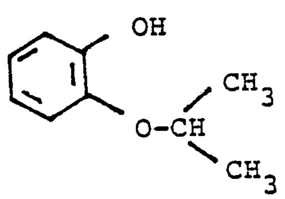

(I)

durch Umsetzung von Brenzkatechin mit Isopropylhalogenid, Aryl- oder Alkylsulfonaten der Formel

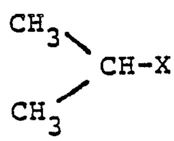

(II)

in welcher X ein Halogenatom oder Alkyl- bzw. Arylsulfonat bedeutet,

in Gegenwart von Alkali- oder Erdalkalibasen bei Temperaturen von 130 °C bis 200 °C in sehr hoher Ausbeute erhalten kann, wenn man in Gegenwart von Polyhydroxyalkyläther mit mindestens einer OH-Gruppe arbeitet und pro Mol Brenzkatechin 1,2 bis 2,0 Mol Alkylierungsmittel und 1,0-2,0 Mol Basenäquivalente einsetzt.

Es ist als ausgesprochen überraschend zu bezeichnen, daß durch die Wahl von Polyhydroxyalkyläthern mit mindestens einer freien OH-Gruppe als Verdünnungsmittel der Brenzkatechinmonoisopropyläther in hohen Ausbeuten bei relativ kurzen Reaktionszeiten erhalten wird. Die hohen Ausbeuten werden ohne Zusatz eines Cokatalysators erzielt. Trotz Überschuß an Alkylierungsmittel ist die Bildung des entsprechenden Diäthers gering. Ein hoher bis quantitativer Brenzkatechinumsatz ist somit bei hoher Selektivität der Monoisopropylätherbildung möglich. Die Nebenreaktionen durch Kernalkylierung sind äußerst gering.

Das erfindungsgemäße Verfahren zeichnet sich durch eine besonders einfache Aufarbeitung aus. Nach Filtration der entstehenden Salze wird die erhaltene organische Phase aufdestilliert. Man erhält dabei nicht nur das verwendete Lösungsmittel zurück, sondern erreicht gleichzeitig durch weitere Destillation die Isolierung des gebildeten Monoäthers in hoher Reinheit.

Der Reaktionsverlauf kann durch folgendes Formelschema wiedergegeben werden :

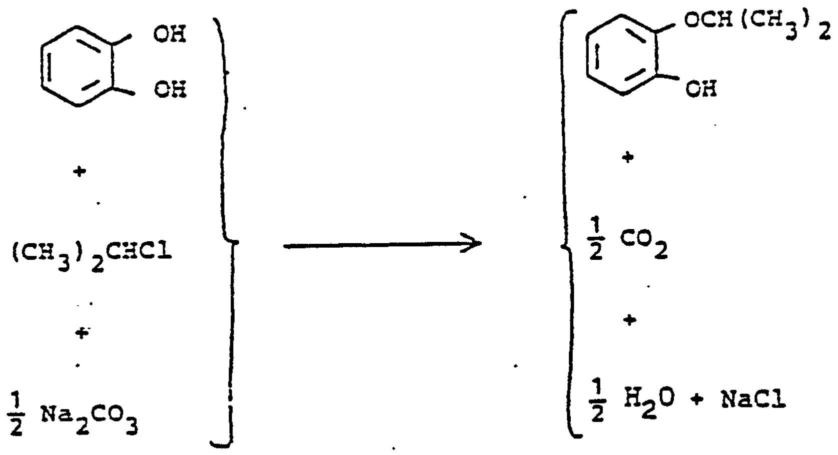

Bei der Durchführung des erfindungsgemäßen Verfahrens läßt man 1 Mol Brenzkatechin mit 1,2-2,0 Mol Alkylierungsmittel der Formel (II) reagieren. Bevorzugt ist das Verhältnis 1,5-1,8 Mol Alkylierungsmittel (II) pro Mol Brenzkatechin. Das Verdünnungsmittel wird im Verhältnis 1,0-4,0 Gewichtsteilen pro Gewichtsteil Brenzkatechin eingesetzt. Günstig ist das Verhältnis 1,5-2,5 kg Verdünnungsmittel pro kg Brenzkatechin.

Es wird in Anwesenheit einer Base gearbeitet. Als solche seien genannt : Alkali- oder Erdalkalihydroxide, -carbonate oder -hydrogencarbonate. Bevorzugt sind Natriumcarbonat oder Natriumhydrogencarbonat und Kaliumcarbonat oder Kaliumhydrogencarbonat. Der Zusatz der Base erfolgt im Verhältnis 1,0-2,0 Basenäquivalente.

Die Umsetzung wird in einem Druckreaktor durchgeführt. Es empfiehlt sich unter Luftausschluß zu arbeiten. Der Reaktionsdruck wird in erster Linie durch das bei der Reaktion freiwerdende $CO_2$ und durch den Siedepunkt des Lösungsmittels bzw. Alkylierungsmittels bestimmt. Das gebildete $CO_2$ kann während der Reaktion kontinuierlich bei erhöhtem Druck entspannt werden.

Es wird bei Temperaturen zwischen 130°-200°C, bevorzugt zwischen 150°-180°C gearbeitet.

Bei den als Verdünnungsmittel verwendeten Polyhydroxyalkyläthern mit mindestens einer freien OH-Gruppe handelt es sich bevorzugt um Glykolmonoalkyläther mit 1-4 C-Atomen in der Alkylgruppe des Alkoxyrestes ; besonders bevorzugt ist der Glykolmonomethyläther.

Das in der Reaktion gebildete oder durch die Einsatzstoffe eingeschleppte Wasser wird bei der Aufarbeitung durch Destillation eines Azeotrops entfernt. Die bei der Reaktion gebildeten Salze bzw. überschüssige Base können im Anschluß an die Reaktion durch Filtration bequem abgetrennt werden. Die erhaltene organische phase liefert bei der Destillation das im Überschuß eingesetzte Alkylierungsmittel, das gebildete Reaktionswasser in Form eines Azeotrops, das eingesetzte Lösungsmittel als Reinkomponente sowie den gebildeten Brenzkatechinmonoisopropyläther in hoher Reinheit und Ausbeute. Die wiedergewonnenen Alkylierungs- und Lösungsmittelmengen werden im nächsten Ansatz ohne weitere Reinigung direkt wieder eingesetzt. Das Produkt Brenzkatechinmonoisopropyläther ist bekannt und findet zur Herstellung von insektiziden Pflanzenschutzmitteln Verwendung.

Beispiel 1

In einem 10 l-VA-Reaktor legt man 896 g Brenzkatechin (8 Mol), 1 157 g Isopropylchlorid techn. (14 Mol), 1 613 g Glykolmonomethyläther und 638 g Natriumcarbonat techn. (6 Mol) vor, spült den Autoklaven mit $N_2$ und heizt unter Rühren auf 170° auf. Der entstehende Druck wird kontinuierlich bei 13-15 bar entspannt. Nach 6 Stunden Reaktionszeit kühlt man auf 20° ab, filtriert vom Salzniederschlag ab, wäscht den Filterrückstand 2 mal mit je 500 g Glykolmonomethyläther und destilliert die erhaltene organische Phase auf. Als 1. Fraktion erhält man bei Normaldruck bei 35°-45° das überschüssige Isopropylchlorid in

hoher Reinheit zurück. Bei der weiteren Destillation bis 105° bei Normaldruck geht ein Azeotrop über, welches das enstandene Reaktionswasser, gebildete Nebenkomponenten und wenig Glykolmonomethyläther enthält. Diese Fraktion wird am zweckmäßigsten verworfen.

Die Fraktion bis ca. 118° bei Normaldruck ist je nach Destillationsbedingungen relativ klein und enthält die restliche Nebenkomponente Methylglykol-isopropyläther zusammen mit dem Lösungsmittel. Das Lösungsmittel wird am zweckmäßigsten bei 20 Torr und einem Siedepunkt über 40° abdestilliert. Man erhält dabei 2 250g reines Methylglykol, welches zusammen mit dem Lösungsmittelgehalt des Salzrückstandes (100 g) eine Wiedergewinnungsrate von 90 % ergibt. Als 5. Fraktion fällt bei 108-110 °C und einem Druck von 20 Torr (2666Pa) das gebildete Isopropoxyphenol in hoher Reinheit mit einer Ausbeute von 81,4 % bezogen auf eingesetztes Brenzkatechin, an. Der Destillationsrückstand wird verworfen. Der Diether-Anteil liegt nach GC-Analyse bei 5 % der Theorie.

Beispiel 2

In einem 0,7 l-VA-Autoklaven werden 56 g Brenzkatechin techn. (0,5 Mol), 62 g Isopropylchlorid techn. (0,75 Mol), 46,4 g Natriumcarbonat techn. (0,44 Mol) und 100 g Glykolmonomethyläther vorgelegt. Der Autoklav wird mit $N_2$ gespült und 5 Stunden bei 170 °C gerührt. Der entstehende Druck ($CO_2$) wird bei 15 bar kontinuierlich entspannt. Nach dem Abkühlen und Entspannen filtriert man den Salzniederschlag ab und wäscht diesen 2 mal mit je 50 ml Lösungsmittel nach. Die so erhaltene organische Phase enthält nach gaschromatographischer Analyse den Monoäther in einer Ausbeute von 76 % der Theorie ; an Diether haben sich 3,4 % der Theorie gebildet.

Beispiel 3

In einem 3 l-VA-Autoklaven werden 337 g Brenzkatechin techn. (3 Mol), 372 g Isopropylchlorid techn. (4,5 Mol), 245 g Soda techn. (2,25 Mol) und 557 g Glykolmonomethyläther vorgelegt. Nach dem Spülen mit $N_2$ heizt man den Autoklaven 6 Stunden bei 170 °C. Es stellt sich ein max. Druck von 29 bar ein. Nach dem Abkühlen und Entspannen wird der Salzniederschlag filtriert und zweimal mit 200 ml Lösungsmittel gewaschen. Die präparative Aufarbeitung analog Beispiel 1 bringt eine Ausbeute von Monoäther von 77 % der Theorie ; an Diether zeigt das GC der organischen Phase eine Ausbeute von 3 %.

Beispiel 4

In einem 0,7 l-VA-Autoklaven legt man 56 g Brenzkatechin techn. (0,5 Mol), 73 g Isopropylchlorid techn. (0,87 Mol), 40 g Soda techn. (0,37 Mol) und 100 g Glykolmonomethyläther vor, spült den Autoklaven kurz mit $N_2$ und rührt die Reaktionsmischung 3 Stunden bei 190°. Es stellt sich ein Druck von 29 bar ein. Nachdem Abkühlen und Entspannen wird der Salzniederschlag abfiltriert, gewaschen und die organische Phase gaschromatographisch analysiert. Man erhält unter diesen Bedingungen den Monoäther in einer Ausbeute von 79 % der Theorie bei einem Dietheranteil von 3,7 % der Theorie.

Beispiel 5

In einem 0,7 l-VA-Autoklaven werden 56 g Brenzkatechin techn. (0,5 Mol), 73 g Isopropylchlorid techn. (0,87 Mol), 46 g Soda techn. (0,43 Mol) und 150 g Glykolmonomethyläther 3 Stunden bei 190° gerührt. Die organische Phase enthält nach Filtration der Salze den Monoäther in einer Ausbeute von 80 % bez. auf Brenzkatechin-Einsatz ; an Diether haben sich 4 % gebildet (GC-Analysen).

Beispiel 6

In einem 10 l-VA-Reaktor legt man 896 g Brenzkatechin techn. (8 Mol), 992 g Isopropylchlorid techn. (12 Mol), 1 485 g Glykolmonomethyläther und 828 g Kaliumcarbonat techn. (6 Mol) vor. Nach dem Spülen des Autoklaven mit $N_2$ heizt man unter Rühren auf 170° auf. Während der Reaktionszeit von 6 Stunden wird der entstehende Druck kontinuierlich bei 15 bar entspannt. Nach dem Abkühlen arbeitet man den Ansatz analog Beispiel 1 auf. Es wird kein überschüssiges Isopropylchlorid mehr gefunden. Die Ausbeute an Isopropoxiphenol liegt bei 77 %;'die Diether-Bildung beträgt laut GC der organischen Phase 12 % der Theorie.

Beispiel 7

In einem 10 l-VA-Autoklaven legt man 896 g Brenzkatechin techn. (8 Mol), 1 157 g Isopropylchlorid techn. (14 Mol), 1 610 g Glykolmonomethyläther und 828 g $K_2CO_3$ (6 Mol) vor. Der Autoklav wird zunächst mit $N_2$ gespült und anschließend 6 Stunden bei 150° gerührt. Es stellt sich ein max. Druck von 22 bar ein. Nach dem Abkühlen wird der Ansatz analog Beispiel 1 aufgearbeitet. Als 1. Fraktion erhält man 215 g Isopropylchlorid (91,6 % vom Einsatz) ; als 5. Fraktion das Isopropoxiphenol in einer Ausbeute von 80,3 %; der Diether fällt nach GC der organischen phase mit einer Ausbeute von 8,2 % der Theorie an.

Beispiel 8

Führt man die Umsetzung analog Beispiel 7, jedoch über 6 Stunden bei 170° durch, so stellt sich ein maximaler Druck von 28 bar ein. Nach dem Abkühlen und Aufarbeiten analog Beispiel 1 gewinnt man zunächst als 1. Fraktion 111 g Isopropylchlorid (10 % vom Einsatz) zurück. Das gebildete Isopropoxiphenol fällt mit hoher Reinheit als 5. Fraktion in einer Ausbeute von 85 % der Theorie an ; der Diether-Anteil (nach Gaschromatographie der organischen phase) liegt bei 9,6 % der Theorie.

Beispiel 9

Arbeitet man analog Beispiel 8, entspannt aber kontinuierlich das gebildete $CO_2$ bei ca. 16 bar, so liegt folgende Produktverteilung nach Destillation der organischen Phase vor : Der Anteil des nicht umgesetzten Isopropylchlorids liegt bei 4,1 % des Einsatzes ; die Ausbeute an Isopropoxiphenol beträgt nach GC der organischen Phase 86 % der Theorie.

**Patentansprüche**

1. Verfahren zur Herstellung von Brenzkatechinmonoisopropyläther der Formel

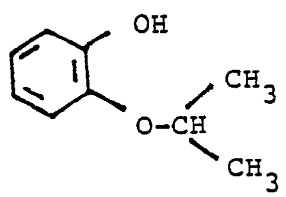

(I)

durch Umsetzung von Brenzkatechin mit Isopropylhalogenid, Aryl- oder Alkylsulfonaten der Formel

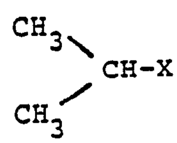

(II)

in welcher X ein Halogenatom oder Alkyl- bzw. Arylsulfonat bedeutet
in Gegenwart von Alkali- oder Erdalkalibasen bei Temperaturen von 130 °C bis 200 °C, dadurch gekennzeichnet, daß man in Gegenwart von Polyhydroxyalkyläther mit mindestens einer OH-Gruppe arbeitet und daß man pro Mol Brenzkatechin 1,2-2,0 Mol Alkylierungsmittel der Formel (II) und 1,0-2,0 Mol Basenäquivalente einsetzt.

2. Verfahren gemäß Anspruch 1, dadurch gekennzeichnet, daß als Verdünnungsmittel Glykolmonomethyläther verwendet wird.

**Claims**

1. Process for the preparation of pyrocatechol monoisopropyl ether of the formula

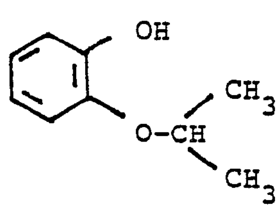

(I)

by reacting pyrocatechol with isopropyl halide or aryl or alkyl sulphonates of the formula

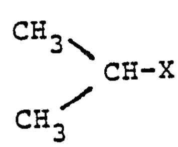

(II)

in which X denotes a halogen atom or alkyl or aryl sulphonate, in the presence of alkali metal or alkaline earth metal bases at temperatures of 130 °C to 200 °C,
characterised in that the reaction is carried out in the presence of polyhydroxyalky ; ether with at least one OH group and in that 1.2-2.0 moles of alkylating agent of the formula (II) and 1.0-2.0 moles of base equivalents are used per mole of pyrocatechol.

2. Process according to Claim 1, characterised in that glycol monomethyl ether is used as the diluent.

**Revendications**

1. Procédé de production d'éther monoisopropylique de pyrocatéchol de formule

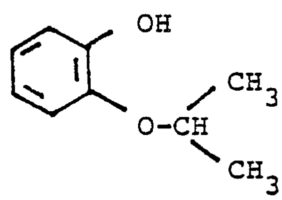

(I)

par réaction de pyrocatéchol avec un halogénure, des aryl- ou des alkylsulfonates d'isopropyle de formule II

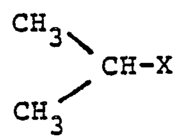

(II)

dans laquelle X est un atome d'halogène ou un groupe alkyl- ou arylsulfonate,
en présence de bases alcalines ou alcalino- terreuses à des températures de 130 à 200 °C, caractérisé en ce qu'on opère en présence d'éther poly-hydroxyalkylique portant au moins un groupe OH et en ce qu'on utilise par mole de pyrocatéchol 1,2-2,0 moles d'agent alkylant de formule (II) et 1,0-2,0 moles d'équivalents de base.

2. Procédé suivant la revendication 1, caractérisé en ce qu'on utilise comme diluant l'éther monométhylique du glycol.